# EUROPÄISCHE PATENTSCHRIFT

(11) **EP 0 345 614 B1**
(45) Veröffentlichungstag und Bekanntmachung des Hinweises auf die Patenterteilung: **22.06.1994**
(21) Anmeldenummer: 89109770.1
(22) Anmeldetag: 30.05.1989
(51) Int. Cl.: C07K 15/08, A61K 37/02, A61K 35/56

(54) **Amblyommin, ein neuer Wirkstoff für die Antikoagulationstherapie**
Amblyommine as an agent for use in antithrombotic therapy
Amblyommine, un agent modifié pour l'usage en thérapie antithrombotique

(30) Priorität: 04.06.1988 DE 3819078
(43) Veröffentlichungstag der Anmeldung: 13.12.1989
(73) Patentinhaber: HOECHST AKTIENGESELLSCHAFT, 65926 Frankfurt am Main (DE)
(72) Erfinder: Bonin, Werner, Dr., D-6233 Kelkheim(Taunus) (DE); Habermann, Paul, Dr., D-6239 Eppstein/Taunus (DE); Tripier, Dominique, Dr., D-6239 Eppstein/Taunus (DE); Wöhner, Elisabet, D-3050 Bunstorf (DE)

(56) Entgegenhaltungen:
- JOURNAL EXP. MED., Band 161, Nr. 2, 1985, The Rockefeller University Press; J.M.C. RIBEIRO et al., Seiten 332-344#
- BIOCHEMICAL JOURNAL, Band 189, 1980, The Biochemical Society (GB); P. WILLADSEN et al., Seiten 295-303#

## Beschreibung

Die Erfindung betrifft den neuen Wirkstoff Amblyommin für die Antikoagulationstherapie, sowie Verfahren zur dessen Isolierung.
Antikoagulantien sind von großer Bedeutung für die Medizin. Als zentrale medizinische Probleme können z.B. die Thromboseprophylaxe, arterielle Reokklusionsprophylaxe, Behandlung von Thrombosen, Verbrauchskoagulopathie und die extracorporale Zirkulation genannt werden.
Die in der Antikoagulationstherapie zur Zeit gebräuchlichen Agentien weisen häufig einige unerwünschte Nachteile wie z.B. Blutungsrisiko, Thrombozytenabfall, Wirkung auf das ZNS, Unverträglichkeit oder indirekte Wirkung auf wie z.B. beim Heparinwirkschema beobachtet wird.
Walsmann und Markwardt (Die Pharmazie Bd. 36 (1981) S. 653) beschreiben die pharmazeutische Bedeutung des aus Hirudo medicinalis gewonnenen Hirudins. Dies ist ein 64-65 Aminosäuren langes Polypeptid (Chan, FEBS Bd. 164 (1983) S. 307; United States Patent 4,668,662). Das Peptid ist ein hochspezifischer Thrombinhemmer, der ansonsten pharmakologisch inert zu sein scheint. Nachteile der Verbindung liegen in der relativ kurzen Halbwertszeit der Verbindung und in der parenteralen Applikationsform.

Wünschenswert ist weiterhin eine antithrombotisch wirksame Verbindung, die keine der genannten Nachteile aufweist. Es ist also sinnvoll nach weiteren Antikoagulantien zu suchen, die die oben genannte medizinische Problematik optimal abdecken.

Schildzecken der Familie Ixodidae sind in allen Entwicklungsstadien temporär, blutsaugende Ektoparasiten von Reptilien, Vögeln, Säugetieren und Menschen. Adulte Zeckenweibchen verweilen gewöhnlich mehrere Tage auf dem Wirt und saugen bis zum Hundertfachen ihres Eigengewichtes an Blut. Dazu besitzen Zecken stechend-saugende Mundgliedmaßen, bestehend aus den paarig angelegten Cheliceren und dem Hypostom. Mit dem Schneidemechanismus der Cheliceren, den Digits, schneiden die Zecken eine Wunde in die Epidermis, in die die Cheliceren selbst und das Hypostom eingeführt werden, womit der Saugkanal gebildet wird. Die Speicheldrüsen der allermeisten Zeckenarten produzieren ein Protein, den sogenannten Zement, das in die Wunde abgegeben wird, und in dem sich die Zähne des Hypostoms verankern und damit die Zecken zur Nahrungsaufnahme am Wirt fixieren.

Weiterhin geben die Zecken ein Sekret ihrer Speicheldrüsen ab, das Antikoagulantien enthält.

Zum Blutsaugen dringen die Mundwerkzeuge der Zecken nicht direkt in Blutkapillaren, sondern in die Gewebe ein. Vermittels der Speicheldrüsenenzyme lysieren sie dort Gewebe und lädieren feinste Kapillaren, aus denen Blut austritt. Durch die gerinnungshemmende Wirkung des Speicheldrüsensekretes bluten die lädierten Gefäße eine Weile und schaffen so eine Gewebsvakuole, aus der die Zecken ihre Nahrung aufnehmen.

Zur Gewinnung von Stoffen mit blutgerinnungshemmenden Eigenschaften wurden deshalb Speicheldrüsen von Zecken untersucht.

Die Erfindung betrifft ein Protein oder ein pharmazeutisch annehmbares Salz davon, das aus Schildzecken isoliert wird und über Thrombininaktivierung die Blutgerinnung hemmt.

Obwohl dieses Protein und Hirudin am selben Substrat angreifen, sind beide Proteine überraschend deutlich voneinander verschieden. Dies läßt erwarten, daß das erfindungsgemäße Protein - genannt Amblyommin - einen von Hirudin verschiedenen medizinischen Stellenwert erhält. Amblyommin ist ein neues Protein. Es ist der erste aus Speicheldrüsen der südafrikanischen Buntzecken isolierte Wirkstoff.

Amblyommin ist durch folgende physikalische Eigenschaften charakterisiert. Es zeigt verhältnismäßig geringe Affinität zu ®Coomassie Brillant Blue R250 Bio-Rad®. Nach Lyophilisation läßt es sich nur schwer in Wasser lösen. Das Protein ist beständig in allen verwendeten Puffern. Friert man das Eluat nach HPLC Reinigung bei -80 °C, so ist keine Aktivitätsminderung feststellbar. Die Verbindung ist ferner stabil bei 40 °C. Sie ist mit Aceton bei pH 1,8 (Essigsäure oder Trichloressigsäure) fällbar. Dabei Wird volle Aktivität der Auflösung der Fällung auch nach mindestens 12 Tagen beobachtet. Verwendet man zur HPLC-Analytik ein Waters 600 Multisolvent Delivery System und eine Biorad RP304 Trennsäule und legt einen Gradienten von 80 % A -20 % B (mit A = 0,1 % TFA und B = 0,1 % TFA in 80 % Acetonitril in HPLC-Wasser) an, wobei nach einer Laufzeit von 40 Minuten 50 % B erreicht ist, so wird für Amblyommin eine Retentionszeit von 27 Minuten gemessen. Mit Gelfiltration über G75 superfine Material wird dem Protein ein Molekulargewicht von 20000-30000 Dalton zugeordnet. In einem SDS-Polyacrylamidgel wird ein Molekulargewicht von 26000 dt ± 2000 dt bestimmt. Die Bestimmung des Isoelektrischen Punktes ergibt einen Wert von ca. 5,35 ± 0,3. Letztlich wurden folgende Teilaminosäuresequenzen 
-Ile-Leu-Phe-Thr-Gln-Gly-Asn-X-Gly-Y-Leu-Glu-Asn-X-Phe-Glu-,
-Lys-Ile-Leu-Phe-X-Y-Gly- und

-Ala-Ser-Tyr-Ile-Val-X-Ser-Glu-Ser-Ile-Gln-Ile-Leu-X-Leu-
Ser-Glu-Gly-Ile-
ermittelt, worin jeder Rest X gleich oder verschieden sein kann und jeweils eine natürlich vorkommende Aminosäure, wie z.B. Tyr, Ser, Thr, Glu, Asp, Asn oder irgendeine nach der Translation modifizierte Aminosäure, wie Trimethyllysin oder γ-Carboxyglutaminsäure, bedeutet und Y Gln oder Glu bedeutet.

Als biologische Eigenschaft ist die spezifische Hemmung von Thrombin herausragend. Das erfindungsgemäße Protein findet daher in der Medizin als Antithrombotikum Anwendung.

### Beispiel 1 Isolation der Zeckendrüsen

Die Speicheldrüse der Zecken ist ein paariges Organ und sitzt in der Sagittalrichtung an der rechten und linken Seite der vorderen Körperhälfte. Je nach Funktionszustand ist die Speicheldrüse klein bei hungernden Exemplaren oder mächtig groß bei saugenden Zecken. Die verwendeten Zecken waren Angehörige der südafrikanischen Buntzecke, Amblyomma hebraeum und Rhipicephalus evertsi, zwei seit Jahren nach bekannten Methoden im Laboratorium gezüchteten Kolonien.

Es wurden teilgesaugte Weibchen im Gewichtsbereich 150-400 mg vom Wirtstier entnommen und unmittelbare darauf unter einem Stereomikroskop seziert, indem die Zecken in einer Petrischale mit der Rückseite auf doppelseitig klebendes Klebeband fixiert und mit PBS-Puffer, pH 7,2, überschichtet wurden. Nach einem Schnitt mittels feinem Skalpell entlang der Peripherie der Zecken wurden die Ober- und Unterseite aufgeklappt und die Speicheldrüsen vorsichtig entnommen und in Stickstoff tiefgefroren.

### Beispiel 2 Extraktion des Wirkstoffes aus Zeckendrüsen

Speicheldrüsen von Zecken der Gattung Amblyomma hebraeum werden isoliert und anschließend unter Stickstoff Zu einem feinen Pulver zermörsert. Das Homogenisat wird in Wasser gelöst und lyophilisiert. Der getrocknete Rückstand wird in 40 %igem Aceton und bei 40 °C 30 Minuten heftig geschüttelt. Anschließend wird bei 15000 g 10 Minuten zentrifugiert. Das Sediment wird wie beschrieben erneut extrahiert und der Überstand mit dem der ersten Extraktion vereinigt und mit 1 M Essigsäure langsam versetzt bis ein pH von 5 eingestellt ist. Die entstehende Fällung wird abzentrifugiert und der klare Überstand abgehebert. Der Überstand wird mit 10 %iger Trichloressigsäure in Aceton versetzt bis ein pH 1,81 erreicht ist. Dann wird das 10fache Volumen eiskaltes Aceton zugegeben und 3,5 Stunden bei -20 °C gefällt. Die Fällung wird 10 Minuten bei 6000 rpm in der Sorvall-Kühlzentrifuge abzentrifugiert. Das Sediment wird mit eiskaltem Aceton und Ether gewaschen und anschließend in Wasser resuspensiert. Die Suspension wird bei -80 °C gefroren und anschließend im Rotationsvakuumverdampfer lyophilisiert. Der getrocknete Rückstand wird in Puffer (0,05 M Triethanolamin-HCl 0,4 M NaCl pH 7,8) aufgenommen.

### Beispiel 3 Gelfiltration

Das in Beispiel 2 dargestellte Proteingemisch wird über Gelfiltration gereinigt. Dazu wird eine Säule von 1,6 cm Durchmesser und 78,3 cm Höhe mit G 75 superfine Material der Firma Pharmazia gefüllt. Die Flußzeit beträgt 10 ml/Stunde. Die Säule wurde mit einem Gelfiltrations-Kalibrierungs-Kit der Firma Pharmacia (Art. No. 17-0442-01) geeicht. Thrombinhemmende Aktivität wird in Fraktionen, die dem Molekulargewichtsbereich 20000-30000 dt zugeordnet werden kann, entdeckt.

### Beispiel 4 Hemmung der Gerinnung von Plasma

Von den einzelnen G 75-Fraktionen werden 50 »l Aliquots mit 50 »l H₂O und 100 »1l Citrat behandeltem Hundeplasma, das 3 U Thrombin pro ml enthielt, gemischt und die Thrombinzeit bestimmt. Die Durchführung erfolgt nach Markwardt et al. (Pharmazie 43 (1988) 202; Fol. Haematol. 115 (1988) 70).

### Beispiel 5 Hemmung der Thrombinkatalysierten Spaltung von Chromozym TH

Die Bestimmung erfolgt gemäß der Publikation von Griesbach et al. (Thromb. Res. 37 (1985), 347-50) Die Methode wird dabei so verändert, daß die Reaktion in 'Mikrotiter Platten-Wells' durchgeführt wird, wobei das Endvolumen 0,2 ml beträgt. die Auswertung erfolgt bei 405 nm mit einem 'Elisa-Reader-Gerät'.

### Beispiel 6 Reinigung mittels Thrombinsäulen-Affinitätschromatographie

Thrombin-Sepharose wird gemäß der Methode von Walsmann, P. (Pharmazie 36 (1981) 860-861) hergestellt. Die G 75-Fraktionen, die Aktivität zeigen, werden vereinigt, lyophilisiert und das Lyophylisat in 0,1 M Essigsäure aufgenommen. Die Proben werden über eine gekühlte G 25-Säule entsalzt und das Eluat gemäß Beispiel 5 auf Aktivität getestet. Aktive Fraktionen werden vereinigt und lyophilisiert. Der getrocknete Rest wird in 0,05 M Tris-HCl (pH 8) aufgenommen und über eine vorbereitete Thrombinsäule gegeben. Alle Schritte werden bei 4 °C durchgeführt. Die Säule wird 2mal mit 0,05 M Tris-Puffer pH 8 gewaschen bevor sie mit 5 M Benzamidiniumchlorid in 0,05 M Tris-HCl (pH 8) eluiert wird. Die Fraktionen werden aufgefangen, Aliquots entnommen, vereinigt und gegen H₂O dialysiert, und auf Thrombinhemmung getestet. Die Fraktionen, die höchste Aktivität zeigen, werden dialysiert und lyophilisiert. Das Lyophylisat wird über SDS-Polyacrylamidgelelektrophorese mit anschließender Silberfärbung unter Verwendung eines 'Färbe-Kits' der Firma Bio-Rad analysiert. Im MW-Bereich von ca. 25000 dt wird eine Proteinbande entdeckt, die bei Elution der Säule ohne vorherige Auftragung der Zeckenprobe nicht beobachtet wird.

### Beispiel 7 Anzucht der Zecken

Die Anzucht der dreiwertigen Zecken der Spezies Rhipicephalus evertsi erfolgt gemäß Th. Hiepe (Lehrbuch der Parasitologie, Bd. 4, Gustav Fischer Verlag Stuttgart 1982, Seite 46 ff.). Die Dauer beträgt ca. 3-4 Monate. Weibliche Zecken werden bei 28 °C und einer Luftfeuchtigkeit größer 90 % zur Eiablage aufbewahrt. Nach dem Schlüpfen der Larven werden diese zur ersten Blutmahlzeit Kaninchen angesetzt. Nach der Mahlzeit erfolgt die Häutung zu Nymphen nüchtern. Diese werden Kaninchen zur zweiten Blutmahlzeit angesetzt. Die vollgesaugten Nymphen treten nun in das Differenzierungsstadium zu adulten Männchen und Weibchen ein.
Nach Abschluß der Differenzierung werden die adulten Tiere verwendet.

### Beispiel 8 Isolierung von Amblyommin aus adulten Zecken

Adulte Zecken werden in H₂O in einem Ultra-Turrax-Gerät der Firma Janke und Kunkel zerkleinert. Das Zeckenhomogenisat wird anschließend 40 Minuten bei 40 °C inkubiert und dann 30 Minuten bei 10000 rpm und 5 °C in einer Sorvall-Zentrifuge zentrifugiert. Das Sediment wird in H₂O resuspendiert und erneut bei 40 °C für 30 Minuten inkubiert und anschließend zentrifugiert. Die Überstände der beiden Zentrifugationsschritte werden vereinigt und durch Blaubandfilter filtriert. Der klare Überstand wird lyophilisiert und der Rückstand in 20 mM Tris-HCl (pH 7,5) aufgenommen und anschließend über Q-Sepharose® Fast Flow Chromatographie (Gradient 0-1 M NaCl in 20 mM Tris-HCl, pH 7,5) gereinigt. Die amblyomminhaltigen Fraktionen werden gemäß Beispiel 5 identifiziert, lyophylisiert und über eine G 75 superfine Säule gemäß Beispiel 3 gegeben. Die antithrombotisch wirksamen Fraktionen eluieren im Molekulargewichtsbereich von 20-30000 dt. Sie werden vereinigt und mittels 'reverse Phase' HPLC über eine Bio-Rad® RP 304-Säule gereinigt. Als Gerät wird das Waters 600 Multisolvent Delivery System verwendet. Folgender Gradient wird gefahren: Start 80 % A - 20 % B (mit A = 0,1 % TFA und B = 0,1 % TFA in 80 % Acetonitril) bis 100 % B in 60 Minuten Laufzeit. Die antithrombotisch aktiven Fraktionen werden vereinigt lyophylisiert und erneut über 'reversed Phase' HPLC gereinigt. Folgender Gradient wird angelegt: Start 80 % A : 20 % B (A = 0,1 % TFA; B = 0,1 % TFA in 80 % Acetonitril). Nach einer Laufzeit von 40 Minuten sind 50 % B erreicht.
Unter diesen Bedingungen ergibt sich für die Amblyommin-Fraktion eine Retentionszeit von 27 Minuten.

### Beispiel 9 Bestimmung des Molekulargewichts von Amblyommin

Die HPLC-Fraktion der Retentionszeit von 27 Minuten wird über ein 17,5 %iges SDS-Polyacrylamidgel analysiert. Als 'Molekulargewichts Marker' wird der 'Elektrophoresis Calibration Kit' der Firma Pharmacia (Art. No. 17-0446-01) verwendet. Amblyommin wandert in diesem System knapp unterhalb der 'Carbon-Anhydrase', die mit einem Molekulargewicht von 30000 dt beschrieben ist. Für Amblyommin ergibt sich ein Molekulargewicht von 26000 ± 2000 dt.

### Beispiel 10 Bestimmung des isoelektronischen Punktes

Gemäß Beispiel 8 isoliertes Amblyommin wird einer Ultradünnschicht-Fokusierung, wie sie Dr. Schulte et al. der Firma LKB Instrument GmbH vorschlagen, unterworfen. Es ergibt sich ein isoelektrischer Punkt von 5,35 ± 0,3.

### Beispiel 11 Proteinsequenzanalyse von Amblyommin

Authentisches von adulten Zecken isoliertes Protein ließ sich in unseren Händen nicht N-terminal sequenzieren. Das vorhandene Material wurde deshalb einer Trypsinspaltung unterzogen. Um eine quantitative Trypsinspaltung zu erlauben, wird das Protein zunächst in einer Carboxymethylierungsreaktion derivatisiert. Dazu wird es in 24 »l Puffer (8 M Guanidinium-HCl, 10 mM Tris, 1 mM EDTA) gelöst und 51 »g DTE in 4 »l Puffer sowie 4,52 »g 2-Mercaptoethanol in 5 »l zugesetzt. Das Reaktionsgemisch wird 1 Stunde bei 37 °C inkubiert und dann mit 8,3 »l einer Jodacetamidlösung (0,25 M Jodacetamid pH 8,0 mit KOH eingestellt) versetzt und 1 Stunde bei Raumtemperatur inkubiert. Dann werden 59 »l H₂O zur Verdünnung zugegeben und nach Zusatz von 400 »l C₂H₅OH nach kräftigem Schütteln gefällt und bei 12000 rpm zentrifugiert.
Dann wird das Sediment mit 100 »l CHCl₃ geschüttelt und mit 300 »l H₂O versetzt und erneut zentrifugiert. Die obere Phase wird mit 300 »l C₂H₅OH gemischt. Das Gemisch wird zentrifugiert und das Sediment zur Trypsinspaltung in 66 »l 0,2 M n-Methylmorpholin (pH 8,5 mit Essigsäure eingestellt) gelöst. Dann werden 5 »l Trypsinlösung (3,3 »g Trypsin in N-Methylmorpholinpuffer) zugesetzt und das Gemisch 2 Stunden bei Raumtemperatur inkubiert.
Das Fragmentgemisch wird nun über einen Mikro-Bore-reversed-Phase HPLC-Schritt getrennt. (Mikro-Bore-Säule: 1 mm x 25 cm, Herst. Firma Braun-Lee).
Dazu wird ein linearer Gradient von 10 % A - 60 % B angelegt (A = 10 % Acetonitril in HPLC-Wasser + 0,1 % TFA; B = 10 % H₂O in Acetonitril + 0,1 % TFA)
Drei Peak-Fraktionen lassen sich gut isolieren. Sie werden einzeln auf Glasfiberfiltern des Gasphasensequencers getrocknet und der Sequenzanalyse unterzogen. Dazu wird der 470 A Gas-Phase-Protein-Sequencer der Firma Applied Biosystems mit online PTH-Identifikationssystem verwendet.

Folgende Teilaminosäursequenzer wurden identifiziert, wobei X als Platzhalter für eine natürlich vorkommende Aminosäure steht und Y als Platzhalter für Gln oder Glu steht.
a) Ile-Leu-Phe-Thr-Gln-Gly-Asn-X-Gly-Y-Leu-Glu-Asn-X-Phe-Glu-
b) Lys-Ile-Leu-Phe-X-Y-Gly-
c)

Ala-Ser-Tyr-Ile-Val-X-Ser-Glu-Ser-Ile-Gln-Ile-Leu-X-
Leu-Ser-Glu-Gly-Ile.

## Patentansprüche (Patentansprüche für folgende(n) Vertragsstaat(en): AT, BE, CH, DE, FR, GB, IT, LI, LU, NL, SE)

1. Protein mit thrombininhibitorischer Wirkung oder ein pharmazeutisch annehmbares Salz davon, dadurch gekennzeichnet, daß das Protein aus Schildzecken isoliert wird und ein Molekulargewicht von 20 000 - 30 000 Dalton aufweist.

2. Protein oder ein pharmazeutisch annehmbares Salz davon gemäß Anspruch 1, dadurch gekennzeichnet, daß das genannte Protein
einen isoelektrischen Punkt zwischen 5,05 und 5,65, und die Aminosäureteilsequenzen 
-Ile-Leu-Phe-Thr-Gln-Gly-Asn-X-Gly-Y-Leu-Glu-Asn-X-Phe-Glu-,
-Lys-Ile-Leu-Phe-X-Y-Gly-
und
- Ala-Ser-Tyr-Ile-Val-X-Ser-Glu-Ser-Ile-Gln-Ile-Leu-X-
Leu-Ser-Glu-Gly-Ile-
 aufweist, worin jeder Rest X gleich oder verschieden sein kann und jeweils für eine natürlich vorkommende Aminosäure steht und Y für Gln oder Glu steht.

3. Protein oder ein pharmazeutisch annehmbares Salz davon, gemäß einem oder mehreren der Ansprüche 1 bis 2, dadurch gekennzeichnet, daß das genannte Protein ein Molekulargewicht von 26000 ± 2000 Dalton hat.

4. Protein oder ein pharmazeutisch annehmbares Salz davon, gemäß einem oder mehreren der Ansprüche 1 bis 3, dadurch gekennzeichnet, daß das genannte Protein einen isoelektrischen Punkt von 5,35 hat.

5. Protein oder ein pharmazeutisch annehmbares Salz davon, gemäß einem oder mehreren der Ansprüche 1 bis 4, dadurch gekennzeichnet, daß das genannte Protein die Aminosäureteilsequenzen 
-Ile-Leu-Phe-Thr-Gln-Gly-Asn-X-Gly-Y-Leu-Glu-Asn-X-Phe-Glu-,
-Lys-Ile-Leu-Phe-X-Y-Gly-
und
- Ala-Ser-Tyr-Ile-Val-X-Ser-Glu-Ser-Ile-Gln-Ile-Leu-X-
Leu-Ser-Glu-Gly-Ile-
 aufweist, worin jeder Rest X gleich oder verschieden sein kann und jeweils für Tyr, Ser, Thr, Gln, Asp, Asn oder irgendeine nach der Translation modifizierte Aminosäure, wie Trimethyllysin oder Carboxyglutaminsäure steht und Y für Gln oder Glu steht

6. Protein oder ein pharmazeutisch annehmbares Salz davon gemäß einem oder mehreren der Ansprüche 1 bis 5, dadurch gekennzeichnet, daß das genannte Protein aus Rhipicephalus evertsi isoliert wird.

7. Protein oder ein pharmazeutisch annehmbares Salz davon gemäß einem oder mehreren der Ansprüche 1 und 3, dadurch gekennzeichnet, daß das genannte Protein aus Amblyomma hebraeum isoliert wird.

8. Verfahren zur Isolierung eines gereinigten Proteins, gemäß einem oder mehreren der Ansprüche 1 bis 6 oder 1, 3 und 7, dadurch gekennzeichnet, daß die Isolierung mit Hilfe einer Kombination von Extraktionsmethoden und chromatographischen Methoden durchgeführt wird, wobei erforderlichenfalls das enzymatische Spalten des Proteins, und erforderlichenfalls das Umwandeln des entstandenen Proteins in seine physiologisch tolerierbaren Salze umfaßt wird.

9. Verwendung eines Proteins, gemäß einem oder mehreren der Ansprüche 1 bis 6 oder 1, 3 und 7 als Arzneimittel.

10. Verwendung eines Proteins, gemäß einem oder mehreren der Ansprüche 1 bis 6 oder 1, 3 und 7 als Antithrombotikum.

## Patentansprüche (Patentansprüche für folgende(n) Vertragsstaat(en): ES, GR)

1. Verfahren zur Herstellung eines Proteins mit thrombininhibitorischer Wirkung oder eines pharmazeutisch annehmbares Salzes davon, dadurch gekennzeichnet, daß das Protein aus Schildzecken isoliert wird und ein Molekulargewicht von 20 000 - 30 000 Dalton aufweist und gegebenenfalls in ein pharmazeutisch annehmbares Salz überfuhrt wird.

2. Verfahren gemäß Anspruch 1, dadurch gekennzeichnet, daß das genannte Protein
einen isoelektrischen Punkt zwischen 5,05 und 5,65, und die Aminosäureteilsequenzen 
-Ile-Leu-Phe-Thr-Gln-Gly-Asn-X-Gly-Y-Leu-Glu-Asn-X-Phe-Glu-,
-Lys-Ile-Leu-Phe-X-Y-Gly- und
- Ala-Ser-Tyr-Ile-Val-X-Ser-Glu-Ser-Ile-Gln-Ile-Leu-X-
Leu-Ser-Glu-Gly-Ile-
 aufweist, worin jeder Rest X gleich oder verschieden sein kann und jeweils für eine natürlich vorkommende Aminosäure steht und Y für Gln oder Glu steht.

3. Verfahren gemäß einem oder mehreren der Ansprüche 1 bis 2, dadurch gekennzeichnet, daß das genannte Protein ein Molekulargewicht von 26000 ± 2000 Dalton hat.

4. Verfahren gemäß einem oder mehreren der Ansprüche 1 bis 3, dadurch gekennzeichnet, daß das genannte Protein einen isoelektrischen Punkt von 5,35 hat.

5. Verfahren gemäß einem oder mehreren der Ansprüche 1 bis 4, dadurch gekennzeichnet, daß das genannte Protein die Aminosäureteilsequenzen 
-Ile-Leu-Phe-Thr-Gln-Gly-Asn-X-Gly-Y-Leu-Glu-Asn-X-Phe-Glu-,
-Lys-Ile-Leu-Phe-X-Y-Gly-          und
 
- Ala-Ser-Tyr-Ile-Val-X-Ser-Glu-Ser-Ile-Gln-Ile-Leu-X-
Leu-Ser-Glu-Gly-Ile-
 aufweist worin jeder Rest X gleich oder verschieden sein kann und jeweils für Tyr, Ser, Thr, Gln, Asp, Asn oder irgendeine nach der Translation modifizierte Aminosäure, wie Trimethyllysin oder Carboxyglutaminsäure steht und Y für Gln oder Glu steht.

6. Verfahren gemäß einem oder mehreren der Ansprüche 1 bis 5, dadurch gekennzeichnet, daß das genannte Protein aus Rhipicephalus evertsi isoliert wird.

7. Verfahren gemäß einem oder mehreren der Ansprüche 1 und 3, dadurch gekennzeichnet, daß das genannte Protein aus Amblyomma hebraeum isoliert wird.

8. Verfahren gemäß einem oder mehreren der Ansprüche 1 bis 6 oder 1, 3 und 7, dadurch gekennzeichnet, daß die Isolierung mit Hilfe einer Kombination von Extraktionsmethoden und chromatographischen Methoden durchgeführt wird, wobei erforderlichenfalls das enzymatische Spalten des Proteins, und erforderlichenfalls das Umwandeln des entstandenen Proteins in seine physiologisch tolerierbaren Salze umfaßt wird.

9. Verwendung eines Proteins, hergestellt gemäß einem oder mehreren der Ansprüche 1 bis 6 oder 1, 3 und 7, als Arzneimittel.

10. Verwendung eines Proteins, hergestellt gemäß einem oder mehreren der Ansprüche 1 bis 6 oder 1, 3 und 7, als Antithrombotikum.

## Claims (Claims for the following Contracting State(s): AT, BE, CH, DE, FR, GB, IT, LI, LU, NL, SE)

1. A protein having a thrombin-inhibitory action or a pharmaceutically acceptable salt thereof, the protein being isolated from hard ticks and having a molecular weight of 20000-30000 dalton.

2. A protein or a pharmaceutically acceptable salt thereof as claimed in claim 1, the said protein having an isoelectric point between 5.05 and 5.65 and the partial amino acid sequences 
-   Ile-Leu-Phe-Thr-Gln-Gly-Asn-X-Gly-Y-Leu-Glu-Asn-
    X-Phe-Glu-,
-   Lys-Ile-Leu-Phe-X-Y-Gly- and
-   Ala-Ser-Tyr-Ile-Val-X-Ser-Glu-Ser-Ile-Gln-Ile-
    Leu-X-Leu-Ser-Glu-Gly-Ile-
 in which each radical X can be identical or different and each represents a naturally occurring amino acid, and Y represents Gln or Glu.

3. A protein or a pharmaceutically acceptable salt thereof, as claimed in one or more of claims 1 to 2, the said protein having a molecular weight of 26000 ± 2000 dalton.

4. A protein or a pharmaceutially acceptable salt thereof, as claimed in one or more of claims 1 to 3, the said protein having an isoelectric point of 5.35.

5. A protein or a pharmaceutically acceptable salt thereof, as claimed in one or more of claims 1 to 4, the said protein having the partial amino acid sequences 
-   Ile-Leu-Phe-Thr-Gln-Gly-Asn-X-Gly-Y-Leu-Glu-Asn-
    X-Phe-Glu-,
-   Lys-Ile-Leu-Phe-X-Y-Gly and
-   Ala-Ser-Tyr-Ile-Val-X-Ser-Glu-Ser-Ile-Gln-Ile-
    Leu-X-Leu-Ser-Glu-Gly-Ile-
 in which each radical X can be identical or different and each represents Tyr, Ser, Thr, Gln, Asp, Asn or any amino acid modified after translation, such as trimethyllysine or carboxyglutamic acid, and Y represents Gln or Glu.

6. A protein or a pharmaceutically acceptable salt thereof as claimed in one or more of claims 1 to 5, the said protein being isolated from Rhipicephalus evertsi.

7. A protein or a pharmaceutically acceptable salt thereof as claimed in one or more of claims 1 and 3, the said protein being isolated from Amblyomma hebraeum.

8. A process for the isolation of a purified protein, as claimed in one or more of claims 1 to 6 or 1, 3 and 7, which comprises the isolation being carried out with the aid of a combination of extraction methods and chromatographic methods, embracing if necessary the enzymatic cleavage of the protein and if necessary the conversion of the resulting protein into its physiologically tolerated salts.

9. The use of a protein as claimed in one or more of claims 1 to 6 or 1, 3 and 7 as a pharmaceutical.

10. The use of a protein as claimed in one or more of claims 1 to 6 or 1, 3 and 7 as an antithrombotic.

## Claims (Claims for the following Contracting State(s): ES, GR)

1. A process for preparing a protein having a thrombin-inhibitory action or a pharmaceutically acceptable salt thereof, which comprises the protein being isolated from hard ticks and having a molecular weight of 20000-30000 dalton and being converted if appropriate into a pharmaceutically acceptable salt.

2. The process as claimed in claim 1, wherein said protein has an isoelectric point between 5.05 and 5.65 and the partial amino acid sequences 
-   Ile-Leu-Phe-Thr-Gln-Gly-Asn-X-Gly-Y-Leu-Glu-Asn-
    X-Phe-Glu-,
-   Lys-Ile-Leu-Phe-X-Y-Gly- and
-   Ala-Ser-Tyr-Ile-Val-X-Ser-Glu-Ser-Ile-Gln-Ile-
    Leu-X-Leu-Ser-Glu-Gly-Ile-
 in which each radical X can be identical or different and each represents a naturally occurring amino acid, and Y represents Gln or Glu.

3. The process as claimed in one or more of claims 1 to 2, wherein said protein has a molecular weight of 26000 ± 2000 dalton.

4. The process as claimed in one or more of claims 1 to 3, wherein said protein has an isoelectric point of 5.35.

5. The process as claimed in one or more of claims 1 to 4, wherein said protein has the partial amino acid sequences 
-   Ile-Leu-Phe-Thr-Gln-Gly-Asn-X-Gly-Y-Leu-Glu-Asn-
    X-Phe-Glu-,
-   Lys-Ile-Leu-Phe-X-Y-Gly and
-   Ala-Ser-Tyr-Ile-Val-X-Ser-Glu-Ser-Ile-Gln-Ile-
    Leu-X-Leu-Ser-Glu-Gly-Ile-
 in which each radical X can be identical or different and each represents Tyr, Ser, Thr, Gln, Asp, Asn or any amino acid modified after translation, such as trimethyllysine or carboxyglutamic acid, and Y represents Gln or Glu.

6. The process as claimed in one or more of claims 1 to 5, wherein said protein is isolated from Rhipicephalus evertsi.

7. The process as claimed in one or more of claims 1 and 3, wherein said protein is isolated from Amblyomma hebraeum.

8. The process as claimed in one or more of claims 1 to 6 or 1, 3 and 7, wherein the isolation is carried out with the aid of a combination of extraction methods and chromatographic methods, embracing if necessary the enzymatic cleavage of the protein and if necessary the conversion of the resulting protein into its physiologically tolerated salts.

9. The use of a protein prepared as claimed in one or more of claims 1 to 6 or 1, 3 and 7 as a pharmaceutical.

10. The use of a protein prepared as claimed in one or more of claims 1 to 6 or 1, 3 and 7 as an antithrombotic.

## Revendications (Revendications pour l'(les) Etat(s) contractant(s) suivant(s): AT, BE, CH, DE, FR, GB, IT, LI, LU, NL, SE)

1. Protéine à activité inhibitrice de la thrombine, ou sel pharmaceutiquement acceptable de celle-ci, caractérisés en ce que la protéine est isolée à partir de tiques et présente une masse moléculaire de 20 000-30 000 daltons.

2. Protéine ou sel pharmaceutiquement acceptable de celle-ci selon la revendication 1, caractérisés en ce que ladite protéine présente un point isoélectrique compris entre 5,05 et 5,65 et les séquences partielles d'aminoacides 
-Ile-Leu-Phe-Thr-Gln-Gly-Asn-X-Gly-Y-Leu-Glu-Asn-X-Phe-Glu-,
-Lys-Ile-Leu-Phe-X-Y-Gly- et
-Ala-Ser-Tyr-Ile-Val-X-Ser-Glu-Ser-Ile-Gln-Ile-Leu-X-Leu-
 Ser-Glu-Gly-Ile-
 dans lesquelles chaque reste X peut être identique ou différent et représente dans chaque cas un aminoacide existant dans la nature, et Y représente Gln ou Glu.

3. Protéine ou sel pharmaceutiquement acceptable de celle-ci, selon une ou plusieurs des revendications 1 et 2, caractérisés en ce que ladite protéine a une masse moléculaire de 26 000 ± 2 000 daltons.

4. Protéine ou sel pharmaceutiquement acceptable de celle-ci, selon une ou plusieurs des revendications 1 à 3, caractérisés en ce que ladite protéine a un point isoélectrique de 5,35.

5. Protéine ou sel pharmaceutiquement acceptable de celle-ci, selon une ou plusieurs des revendications 1 à 4, caractérisés en ce que ladite protéine présente les séquences partielles 
-Ile-Leu-Phe-Thr-Gln-Gly-Asn-X-Gly-Y-Leu-Glu-Asn-X-Phe-Glu-,
-Lys-Ile-Leu-Phe-X-Y-Gly- et
-Ala-Ser-Tyr-Ile-Val-X-Ser-Glu-Ser-Ile-Gln-Ile-Leu-X-Leu-
 Ser-Glu-Gly-Ile-,
 dans lesquelles chaque radical X peut être identique ou différent et représente dans chaque cas Tyr, Ser, Thr, Gln, Asp, Asn ou un aminoacide quelconque modifié après la traduction, tel que la triméthyllysine ou l'acide carboxyglutamique, et Y représente Gln ou Glu.

6. Protéine ou sel pharmaceutiquement acceptable de celle-ci, selon une ou plusieurs des revendications 1 à 5, caractérisés en ce que ladite protéine est isolée à partir de *Rhipicephalins evertsi*.

7. Protéine ou sel pharmaceutiquement acceptable de celle-ci, selon une ou plusieurs des revendications 1 et 3, caractérisés en ce que ladite protéine est isolée à partir d'*Amblyomma hebraeum*.

8. Procédé pour l'isolement d'une protéine purifiée, selon une ou plusieurs des revendications 1 à 6 ou 1, 3 et 7, caractérisé en ce que l'isolement est effectué à l'aide d'une association de méthodes d'extraction et de méthodes chromatographiques, si nécessaire la coupure enzymatique de la protéine, et si nécessaire la conversion de la protéine résultante en un de ses sels physiologiquement acceptables étant englobées.

9. Utilisation d'une protéine selon une ou plusieurs des revendications 1 à 6 ou 1, 3 et 7, en tant que médicament.

10. Utilisation d'une protéine selon une ou plusieurs des revendications 1 à 6 ou 1, 3 et 7, en tant qu'agent antithrombique.

## Revendications (Revendications pour l'(les) Etat(s) contractant(s) suivant(s): ES, GR)

1. Procédé pour la préparation d'une protéine à activité inhibitrice de la thrombine, ou d'un sel pharmaceutiquement acceptable de celle-ci, caractérisé en ce que la protéine est isolée à partir de tiques et présente une masse moléculaire de 20 000-30 000 daltons, et est éventuellement convertie en un sel pharmaceutiquement acceptable.

2. Procédé selon la revendication 1, caractérisé en ce que ladite protéine présente un point isoélectrique compris entre 5,05 et 5,65 et les séquences partielles d'aminoacides 
-Ile-Leu-Phe-Thr-Gln-Gly-Asn-X-Gly-Y-Leu-Glu-Asn-X-Phe-Glu-,
-Lys-Ile-Leu-Phe-X-Y-Gly- et
-Ala-Ser-Tyr-Ile-Val-X-Ser-Glu-Ser-Ile-Gln-Ile-Leu-X-Leu-
 Ser-Glu-Gly-Ile-
 dans lesquelles chaque reste X peut être identique ou différent et représente dans chaque cas un aminoacide existant dans la nature, et Y représente Gln ou Glu.

3. Procédé selon une ou plusieurs des revendications 1 et 2, caractérisé en ce que ladite protéine a une masse moléculaire de 26 000 ± 2 000 daltons.

4. Procédé selon une ou plusieurs des revendications 1 à 3, caractérisé en ce que ladite protéine a un point isoélectrique de 5,35.

5. Procédé selon une ou plusieurs des revendications 1 à 4, caractérisé en ce que ladite protéine présente les séquences partielles 
-Ile-Leu-Phe-Thr-Gln-Gly-Asn-X-Gly-Y-Leu-Glu-Asn-X-Phe-Glu-,
-Lys-Ile-Leu-Phe-X-Y-Gly- et
-Ala-Ser-Tyr-Ile-Val-X-Ser-Glu-Ser-Ile-Gln-Ile-Leu-X-Leu-
 Ser-Glu-Gly-Ile-,
 dans lesquelles chaque radical X peut être identique ou différent et représente dans chaque cas Tyr, Ser, Thr, Gln, Asp, Asn ou un aminoacide quelconque modifié après la traduction, tel que la triméthyllysine ou l'acide carboxyglutamique, et Y représente Gln ou Glu.

6. Procédé selon une ou plusieurs des revendications 1 à 5, caractérisé en ce que ladite protéine est isolée à partir de *Rhipicephalus evertsi*.

7. Procédé selon une ou plusieurs des revendications 1 et 3, caractérisé en ce que ladite protéine est isolée à prtir d'*Amblyomma hebraeum*.

8. Procédé selon une ou plusieurs des revendications 1 à 6 ou 1, 3 et 7, caractérisé en ce que l'isolement est effectué à l'aide d'une association de méthodes d'extraction et de méthodes chromatographiques, si nécessaire la coupure enzymatique de la protéine, et si nécessaire la conversion de la protéine résultante en un de ses sels physiologiquement acceptables étant englobées.

9. Utilisation d'une protéine préparée selon une ou plusieurs des revendications 1 à 6 ou 1, 3 et 7, en tant que médicament.

10. Utilisation d'une protéine préparée selon une ou plusieurs des revendications 1 à 6 ou 1, 3 et 7, en tant qu'agent antithrombique.
